# EUROPEAN PATENT APPLICATION

(11) **EP 2 301 514 A2**
(43) Date of publication of application: **30.03.2011**
(21) Application number: 10011256.4
(22) Date of filing: 28.09.2010
(51) Int. Cl.: A61K 6/00, C09J 183/04, C09J 183/10, A61K 6/093

(54) **Adhesive agent for silicone rubber**

(30) Priority: 28.09.2009 JP 2009222980
(71) Applicant: GC Corporation, Itabashi-ku Tokyo 174-8585 (JP)
(72) Inventor: Kamohara, Hiroshi, Tokyo (JP)
(74) Representative: Müller-Boré & Partner Patentanwälte

(57) **Abstract**

An adhesive agent for a silicone rubber used at a time of adhering a silicone rubber which is used for an impression making or a denture relining in a dental care to an impression tray or a denture is made to have a higher initial adhesion property, to dry earlier after being applied, and to have a higher safety for a living body, in comparison with conventional adhesive agents of the same kind. The adhesive agent for a silicone rubber includes (A) methyl acetate in an amount of 99 to 40 weight %, and (B) one kind or two kinds or more of adhesive imparting agents selected from a silicone resin, a silicone raw rubber, a silicone denatured acrylic resin, a silane coupling agent, an organopolysiloxane having an alkenyl group, an organopolysiloxane having at least two hydrogen atoms in one molecule, and a silicone soluble platinum compound in an amount of 1 to 60 weight %. Further, it may include (C) one kind or more of a coloring agent, an antioxidant and a fragrance in an amount of 0.1 to 3 weight %.

## Description

The present invention relates to an adhesive agent used at a time of adhering a silicone rubber which is used for an impression making or a denture relining in a dental care to an impression tray or a denture, and more particularly to an adhesive agent for a dental silicone rubber which has a higher initial adhesion property, dries earlier after being applied, and has a higher safety for a living body, in comparison with a conventional adhesive agent of the same kind.

In the dental care, there is a case that a material, which is cured at a room temperature and a silicone rubber in a paste form is used as a base material, is used for impression making for the purpose of molding an inside of a mouth cavity and denture relining for the purpose of installing to a mucosal surface of a denture.

Among them, in an impression making method for the purpose of molding the inside of the mouth cavity, the silicone rubber paste before curing is mounted on the impression tray, is inserted into the mouth cavity, and is taken away together with the impression tray from the mouth cavity after the silicone rubber paste is cured, however, since it is necessary that the cured silicone rubber impression material is securely retained in the impression tray, there is often the case that the adhesive agent is used. However, at a time of taking away the silicone rubber impression material together with the impression tray from the mouth cavity, there is a case that such a problem that the cured silicone rubber impression material peels off from the impression tray is caused, and there occurs such a problem that an accurate impression of the mouth cavity can not be obtained. The phenomenon mentioned above is a phenomenon caused in the case that a solvent in the adhesive agent after applying of the adhesive agent remains much between the impression tray and the silicone rubber impression material so that an effect of the adhesive agent can not be sufficiently obtained, and the case that generation of adhesive force serving as the adhesive agent is slow, and the silicone rubber impression material is taken away from the mouth cavity before the adhesive force between the impression tray and the silicone rubber impression material rises sufficiently.

On the other hand, there is a case that a silicone rubber in a paste form is used for the denture relining in the same manner as the impression making time for the purpose of reducing a pain of a mucosa in the case that a bulge of bone is very acute and the case that the mouth cavity mucosa becomes very thin, among denture installation patients. In this case, the adhesive agent is applied in the same manner as the impression making time to the mucosal surface of the denture, and is dried, the silicone rubber in the paste form is thereafter mounted on the mucosal surface of the denture to be inserted into the mouth cavity, and is cured, and a cured body of the silicone rubber having a suitable thickness is relined to the mucosal surface of the denture. In this making work, after the silicone rubber is cured, there is carried out an operation for taking out the denture from the mouth cavity of the patient and removing the cured silicone rubber by an exclusive cutting and polishing material for correcting a shape as well as removing a surplus portion of the silicone rubber, however, since a lot of solvent in the adhesive agent after applying of the adhesive agent remains between the mucosal surface of the denture and the silicone rubber in the same manner at the impression making time, the effect serving as the adhesive agent can not be sufficiently obtained, and the generation of the adhesive force serving as the adhesive agent is slow, the cutting and polishing work is carried out before the adhesive force between the silicone rubber and the mucosal surface of the denture rises sufficiently, so that there is caused such a problem that the silicone rubber peels off from the mucosal surface of the denture, the pain can not be sufficiently reduced, and a deterioration in the mouth cavity becomes severe.

As an adhesive agent composition of the silicone rubber in a dentistry as mentioned above, there is disclosed composition made by 1,3 dioxolane and/or a dimethyl carbonate as a solvent, and one kind or two kinds or more selected from a silicone resin, a silane coupling agent, an organopolysiloxane having an alkenyl group, an organopolysiloxane having at least two hydrogen atoms in one molecule, and a silicone denatured acrylic resin as an adhesive imparting agent (refer, for example, to Japanese Unexamined Patent Publication No. 2002-53418). However, it has not taken into consideration the problem in an initial stage of the adhesion, and has not solve the problem caused by the initial low adhesive property at the impression making time and the denture relining time. Further, there is disclosed an adhesive agent including a (meth)acrylic polymer obtained by modifying (a) a peroxide such as a benzoyl peroxide or the like by (b) a siloxane having a reactive functional group for a hydrosilylation reaction such as a Si-H group or the like and an organic solvent such as (c) an ethyl acetate or the like at respective specific rates, for the purpose of improving an adhesion durability as a denture relining material for adhering a silicone composition material and a (meth) acrylic resin (refer, for example, to Japanese Unexamined Patent Publication No. 2004-196956). However, it has not made a study of the improvement of the early adhesive property too, and the problem such as the peeling in the early stage of the adhesion has not been solved.

Accordingly, an object of the present invention is to provide an adhesive agent for a silicone rubber which has a higher initial adhesion property, dries earlier after being applied, and has a higher safety for a living body, at a time of adhering the silicone rubber used for an impression making or a denture relining to an impression tray or a denture.

As a result of devoting to make a study for solving the problem mentioned above, the inventor of the present invention has found the fact that the problem mentioned above can be solved by using both one kind or two kinds or more of adhesive imparting agents selected from a silicone resin, a silicone raw rubber, a silicone denatured acrylic resin, a silane coupling agent, an organopolysiloxane having an alkenyl group, an organopolysiloxane having at least two hydrogen atoms in one molecule and a silicone soluble platinum compound which are used as the adhesive imparting agent, and a methyl acetate as a solvent, and has finished the present invention.

In other words, in accordance with the present invention, there is provided an adhesive agent for a silicone rubber comprising:
(A) methyl acetate in an amount of 99 to 40 weight %; and
(B) one kind or two kinds or more of adhesive imparting agents selected from a silicone resin, a silicone raw rubber, a silicone denatured acrylic resin, a silane coupling agent, an organopolysiloxane having an alkenyl group, an organopolysiloxane having at least two hydrogen atoms in one molecule, and a silicone soluble platinum compound in an amount of 1 to 60 weight %.

Further, the adhesive agent for the silicone rubber may include (C) one kind or more of a coloring agent, an antioxidant and a fragrance in an amount of 0.1 to 3 weight %.

The adhesive agent for a silicone rubber in accordance with the present invention is an adhesive agent for a silicone rubber which has a higher initial adhesion property, is earlier dried after being applied, and has a higher safety for a living body, at a time of adhering a silicone rubber used for impression making or denture relining to an impression tray or a denture.

The methyl acetate corresponding to the component (A) used in the adhesive agent for a silicone rubber in accordance with the present invention is a solvent for dissolving and dispersing an adhesive imparting agent mentioned below. An ethyl acetate is known as a solvent of the same kind, and is used as a solvent in a lot of adhesive agents. However, the inventors of the present invention have first found the fact that the initial adhesion properly is significantly improved by using the methyl acetate in place of the ethyl acetate which has been conventionally used as the solvent of the adhesive imparting agent, and the problem such as the peeling in the early stage of adhesion can be accordingly improved. Since a boiling point of the methyl acetate is lower than that of the ethyl acetate, drying after application is faster in the case that the methyl acetate is used as the solvent. However, this drying speed and the initial adhesion state become significant on the basis of a combination with the adhesive imparting agent. Further, in comparison with a chlorine solvent which has been widely used as the solvent for the adhesive imparting agent having faster volatilization and drying speeds, an adverse effect for an environment and a living body is small, and the methyl acetate can be used as an alternative of the chlorine solvent in this meaning, too. A blending amount of the methyl acetate corresponding to the component (A) is between 99 and 40 weight %. If it is more than 99 weight %, the effect of the adhesive imparting agent corresponding to the component (B) is not sufficient, and if it is less than 40 weight %, a viscosity of the adhesive agent becomes too high, so that the adhesive agent is hard to be applied, and such is not proper. It is preferably between 96 and 70 weight %.

The adhesive imparting agent corresponding to the component (B) used in the present invention is one kind or two kinds or more selected from the silicone resin, the silicone raw rubber, the silicone denatured acrylic resin, the silane coupling agent, the organopolysiloxane having the alkenyl group, the organopolysiloxane having at least two hydrogen atoms in one molecule, and the silicone soluble platinum compound.

The silicone resin is a polymer having a three-dimensional network while having a siloxane bond as a structural unit. The silicone raw rubber is a straight chain organopolysiloxane having an average molecular weight between 400,000 and 800,000, and an alkenyl group, a methyl group, a phenyl group and the like can be exemplified as an organic group included in this. The silicone denatured acrylic resin is an acrylic acid (or a methacrylic acid) alkyl polymer having an organic group including the siloxane bond. The compounds exemplified in the Japanese Unexamined Patent Publication No. 2002-53418 or the like can be used as the silicone denatured acrylic resin, and there can be exemplified an acrylic acid alkyl polymer having an organic group including the siloxane bond, for example, a silicone denatured acrylic resin having a structure as shown in the following chemical formula 1. Needless to say, the alkyl group of the alkyl polymer in the formula can be replaced by an ethyl group, a propyl group, a butyl group and the like without being limited to the methyl group, as well as it is possible to change a connection position between the organic group having the siloxane bond and the acrylic acid alkyl polymer and the number thereof in the chemical formula 1.
[Chemical Formula 1] 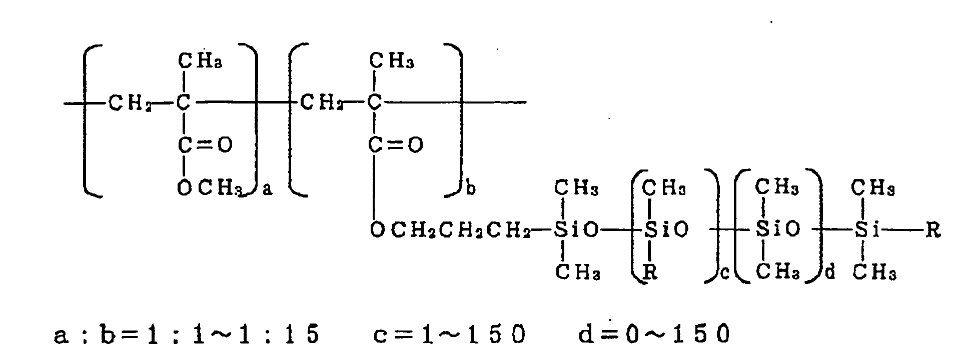 R = hydrogen atom or allyl group

The silane coupling agent can be a silane compound including a vinyl group, a glycidyl group, an amino group, a methacrylate group, an isocyanate group and the like. Further, it can be an organopolysiloxane having an alkenyl group corresponding to a base material of an addition type silicone rubber, and an organopolysiloxane having at least two hydrogen atoms in one molecule corresponding to a cross linking agent of the addition type silicone rubber as well.

Further, it is possible to use a silicone soluble platinum corresponding to a curing catalyst of an additional reaction type silicone rubber, and it is possible to list up a chloroplatinic acid, an alcohol denatured chloroplatinic acid, a complex of a chloroplatinic acid and an olefin which are known additional reaction catalysts and the like as the silicone soluble platinum compound. A vinyl siloxane complex of the chloroplatinic acid is particularly preferably used. In the case of using the silicone soluble platinum compound, it is desirable to use it together with the organopolysiloxane having the alkenyl group, and it is desirable not to use it together with the organopolysiloxane having at least two hydrogen atomics in one molecule. A blending amount of the components (B) is between 1 and 60 weight %. If it is less than 1 %, an adhesive property with the silicone rubber is not sufficient, and if it is more than 60 %, a viscosity of the adhesive agent becomes too high, so that it is hard to be applied.

In the adhesive agent for a silicone rubber in accordance with the present invention, one kind or more of the coloring agent of the inorganic pigment or the organic pigment, the antioxidant and the fragrance may be used in a range between 0.1 and 3 weight %, as the component (C) in a range that the characteristic is not lost.

Particularly exemplifying the inorganic pigment, there can be listed up a chromate salt such as a chrome yellow, a zinc lead, a barium yellow or the like, a ferrocyanide such as an iron blue or the like, a sulfide such as a silver red, a cadmium yellow, a zinc sulfide, an antimony white, a cadmium red or the like, a sulfate such as a barium sulfate, a zinc sulfate, a strontium sulfate or the like, an oxide such as a zinc flower, a titanium white, a red oxide, a black iron oxide, a chrome oxide or the like, a hydroxide such as an aluminum hydroxide or the like, a silicate such as a calcium silicate, an ultramarine blue pigment, or the like, a carbon such as a carbon black, a graphite or the like, and the like. Particularly exemplifying the organic pigment, there can be listed up a nitroso pigment such as a naphthol green B, a naphthol green Y or the like, a nitro pigment such as a naphthol S, a lithol fast yellow 2G or the like, an insoluble azo pigment such as a permanent red 4R, a brilliant fast scarlet, a Hanza yellow, a benzidine yellow or the like, a semi-insoluble azo pigment such as a resole red, a lake red C, a lake red D or the like, a soluble azo pigment such as a brilliant carmine 6B, a permanent red F5R, a pigment scarlet 3B, a bordeaux 10B or the like, a phthalocyanine pigment such as a phthalocyanine blue, a phthalocyanine green, a sky blue or the like, a basic dye pigment such as a rhodamine lake, a malachite green lake, a methyl violet lake or the like, an acid dye pigment such as a peacock blue lake, an eosin lake, a quinoline yellow lake or the like, and the like. These pigments can be used independently or by combining a plurality of pigments.

As the antioxidant, there can be listed up all vitamins A including a retinol and 3,4-didehydro retinol, all carotenes including an α -carotene, a β -carotene, a γ - carotene and a δ -carotene, all vitamins C including a D-ascorbic acid and an L-ascorbic acid, all vitamins E including a vitamin E esters which are easily hydrolyzed so as to become a vitamin E and includes an α -tocopherol, a β -tocopherol, a γ -tocopherol, a δ -tocopherol, a tocoquinone, a tocotrienol, a vitamin E acetate and a vitamin E succinate, and including vitamin E salts which can be allowed pharmacologically such as a vitamin E phosphate, prodrugs of a vitamin A, a carotene, a vitamin C and a vitamin E, and salts of a vitamin A, a carotene, a vitamin C and a vitamin E which can be allowed pharmacologically.

As for the fragrance, for example, there are natural fragrant powder including a freeze dried natural vegetable component, an essential oil, an essence, an extractive and an acid and a synthetic perfume (including the natural fragrance). As an example of liquid and powder fragrances, there can be listed up fruit essences such as a coconut, a coffee, a chocolate, a vanilla, a grapefruit, an orange, a lime, a menthol, a glycyrrhiza glabra, a caramel fragrant substance, a honey fragrant substance, a peanut, a walnut, a cashew, a cobnut, an almond, a pineapple, a strawberry, a raspberry, a tropical fruit, a cherry, a cinnamon, a peppermint, a checkerberry, a spearmint, a eucalyptus, a mint, an apple, a pear, a peach, a strawberry, an apricot, a raspberry, a cherry, a pineapple and a plum essence. As for the essential oil, there can be listed up a peppermint, a spearmint, a menthol, an eucalyptus, a clove oil, a bay oil, an anise, a sime, an arbor vitae oil, a nutmeg and oils of the fruits mentioned above.

A description will be given below of the present invention with reference to embodiments, however, the present invention is not limited to them.

### <Embodiment 1>

An adhesive agent for a silicone having a composition shown in Table 1 was produced.

### <Confirmation of drying speed>

With regard to an influence given to a denture plate by applying of the adhesive agent, a resin for the denture plate (trade name: AKRON, manufactured by GC Corporation. ) was formed into 3 x 3 x 2 mm, the adhesive agent mentioned above was thereafter applied to a primer for a silicone soft relining material (trade name: GC RELINE manufactured by GC Corporation.) by using an accessory brush, and a time, in which a liquid content was evaporated and dryness was recognized visually, was measured. A result thereof is shown in Table 1.

### <Confirmation of initial adhesive property>

A flat plate having a dimension of 60 x 60 x 2 mm was produced by a resin for a denture plate similarly to the above, and the adhesive agent having the composition mentioned above was applied to one third portion from one end and is dried. Thereafter, a silicone relining material (trade name: GC RELINE EXTRASOFT manufactured by GC Corporation.) was kneaded and then build up on a whole surface of the flat plate, was pressed from an upper surface, was dipped into a warm water at 37 °C for five minutes and for twenty four hours, and was formed into a cured body having a thickness of 2 mm, the cured body was immediately cut to have a width of about 10 mm, the other end to which the adhesive agent was not applied was folded back at 180 degree, and a peeling test was carried out by a universal tester (trade name: AUTOGRAPH manufactured by Shimadzu Corp.). A result thereof is shown in Table 1.

**[Table 1]**

| | | EMBODIMENT | | | | | COMPARATIVE EXAMPLE | | | |
|---|---|---|---|---|---|---|---|---|---|---|
| | | 1 | 2 | 3 | 4 | 5 | 1 | 2 | 3 | 4 |
| A | Methyl acetate | 90 | 75 | 84 | 80 | 94 | | | | |
| B | Silicone resin | | 5 | | | | | 5 | | |
| | Silane coupling agent | | | | | | 10 | | | |
| | Silane compound having isocyanate group | | 1 | | | | | | | |
| | Silicone raw rubber | | | | 2 | 4 | | | 10 | |
| | Organopolysiloxane having alkenyl group | | | | | 1 | | | | |
| | Organopolysiloxane having at least two hydrogen atoms in one molecule | | | | | | | | | 10 |
| | Silicone denatured acrylic resin | | | | | | | | | |
| | Acrylic resin including dimethyl siloxane having one SiH in molecule | 10 | 19 | 16 | 18 | | | 20 | | |
| | Silicone soluble platinum compound | | | | | 1 | | | | |
| Ethyl acetate | | | | | | | 90 | | | |
| Butyl acetate | | | | | | | | | 90 | |
| Toluene | | | | | | | | 75 | | |
| Acetone | | | | | | | | | | 90 |
| Drying speed (sec) | | 4 | 6 | 5 | 8 | 8 | 15 | 20 | 40 | 14 |
| Initial adhesive | After 5 minutes | 10.3 | 10.1 | 10.2 | 12 | 8 | 4.1 | 4.0 | 3.2 | 2.8 |
| property (N/mm) | After 24 hours | 11.1 | 10.9 | 10.3 | 12 | 8 | 10.2 | 9.9 | 6.1 | 4.5 |

As shown in Table 1, it is known that the adhesive agent for a silicone rubber in which the methyl acetate is used as the solvent in accordance with the present invention has a high initial adhesive property in spite that the same adhesive imparting agent as the conventional one is used. Further, the drying speed is extremely higher than comparative examples in which other solvents are used. This characteristic expresses that it can prevent peeling between a silicone soft relining material and a denture and peeling between a silicone impression material and an impressing tray which have conventionally been caused by a low initial adhesive property in question.

## Claims

1. An adhesive agent for a silicone rubber comprising:
(A) methyl acetate in an amount of 99 to 40 weight %; and
(B) one kind or two kinds or more of adhesive imparting agents selected from a silicone resin, a silicone raw rubber, a silicone denatured acrylic resin, a silane coupling agent, an organopolysiloxane having an alkenyl group, an organopolysiloxane having at least two hydrogen atoms in one molecule, and a silicone soluble platinum compound in an amount of 1 to 60 weight %.

2. The adhesive agent for a silicone rubber as claimed in claim 1, wherein the adhesive agent for a silicone rubber further includes (C) one kind or more of a coloring agent, an antioxidant and a fragrance in an amount of 0.1 to 3 weight %.
